# EUROPEAN PATENT APPLICATION

(11) **EP 2 901 962 A1**
(43) Date of publication of application: **05.08.2015**
(21) Application number: 13842262.1
(22) Date of filing: 22.07.2013
(51) Int. Cl.: A61C 8/00, A61N 2/08

(54) **MAGNETIC FIELD GENERATION DEVICE FOR PROMOTING BONE GROWTH, IMPLANT DEVICE USING SAME, AND DENTAL PROSTHESIS FOR PROTECTING IMPLANT**

(30) Priority: 25.09.2012 JP 2012211330
(71) Applicant: Aichi Steel Corporation, Tokai-shi, Aichi 476-8666 (JP)
(72) Inventor: ARAI Kazuo, Tokai-shi Aichi 476-8666 (JP); HONKURA Yoshinobu, Tokai-shi Aichi 476-8666 (JP)
(74) Representative: Kramer Barske Schmidtchen Patentanwälte PartG mbB
(86) International application number: PCT/JP2013/069754
(87) International publication number: WO 2014/050271

(57) **Abstract**

A magnetic field generator 3 is attachable to an implant body 2 formed from a non-magnetic material to be inserted in jawbone 61. The magnetic field generator 3 includes a magnet body 30 that generates magnetism. The magnetic field generator 3 is mountable to and removable from the implant body 2. The magnetic field generator 3 in a state of being mounted to the implant body 2 forms a magnetic field around the implant body 2.

## Description

### TECHNICAL FIELD

The invention relates to a magnetic field generator that promotes growth of jawbone and consequently reduces duration of osseointegration, and an implant device and a denture for implant protection using the magnetic field generator.

### BACKGROUND ART

In the field of dentistry, an example of known structures for fixing a denture in oral cavity is an implant-supported structure (Patent Document 1). The implant-supported structure can stably fix the denture in oral cavity by fixing the denture to an implant inserted in alveolar ridge.

An example of such implants is formed from a titanium alloy and has a threaded portion formed on an outer peripheral side surface thereof to be engaged with jawbone . This titanium alloy implant has a roughened surface with very fine projections and dents formed thereon.

The surgical procedures to bury an implant in jawbone are as follows: an incision is made into gingiva of alveolar ridge to form a prepared hole in jawbone, and a threaded hole is formed in an inner peripheral surface of the prepared hole by tapping. Then, a cylindrical closed-bottom implant with threads formed on its outer peripheral side surface is screwed into the threaded hole of jawbone to be planted therein. In about three to six months after the implant is planted in the threaded hole, the implant and jawbone are integrated with each other.

This integral connection between the implant and jawbone is made by osseointegration. The term "osseointegration" refers to the connection of jawbone with titanium used as the material of the implant without immunological rejection. The jawbone with the implant inserted therein grows, narrowing a gap between the implant surface and jawbone and then filling in the fine projections and dents on the implant surface. Then, the implant and jawbone are finally firmly connected to each other.

It is only after the implant-jawbone firm connection is finalized that the denture can be fixed to the implant.

### PRIOR ART DOCUMENT

### PATENT DOCUMENT

Patent Document 1: JP-A-H07-136190

### SUMMARY OF THE INVENTION

### PROBLEMS TO BE SOLVED BY THE INVENTION

The conventional implant device disclosed in the document, however, has the following disadvantages.

It needs a long duration of retention, three to six months, before osseointegration between jawbone and the implant inserted in alveolar ridge is completed. During this period of time, the implant yet to be firmly connected to jawbone is not ready for use as a denture fixing means. While the osseointegration is progressing, therefore, the denture may be arranged directly on alveolar ridge, causing the denture to easily come off. Since a patient is compelled to deal with such inconvenience during the ongoing osseointegration, it is desired to reduce the duration of osseointegration.

The invention was accomplished under the current circumstances. The invention has an object to provide a magnetic field generator that promotes growth of jawbone and consequently reduces duration of osseointegration, and an implant device and a denture for implant protection using the magnetic field generator.

### SOLUTIONS TO THE PROBLEMS

One aspect of the present invention resides in a magnetic field generator attachable to an implant body formed from a non-magnetic material to be inserted in jawbone including at least one magnet body that generates magnetism, wherein
the magnetic field generator is mountable to and removable from the implant body, and
the magnetic field generator in a state of being mounted to the implant body forms a magnetic field around the implant body.

Another aspect of the present invention resides in an implant device, including:
the magnetic field generator; and
an implant body formed from a non-magnetic material to be inserted in jawbone, wherein
the magnetic field generator is mountable to and removable from the implant body.

Further another aspect of the present invention resides in a denture for implant protection mounted on alveolar ridge mounted with the implant device, including:
a denture base for covering the alveolar ridge; and
an artificial tooth formed on the denture base, wherein
the denture base has a protective concave portion that, in a non-contact manner, accommodates therein a part of the implant device protruding from the jawbone, and
the denture base includes a magnetic member that induces a magnetic field from the magnetic field generator.

### EFFECTS OF THE INVENTION

As described above, the magnetic field generator is configured to form a magnetic field around the implant body. Thereby, it is considered that the duration of osseointegration may be reduced.

A well-accepted practice for effective bone growth in the field of orthopedics is to impart magnetic stimulation to osteoblasts through a magnetic field to promote growth of the osteoblasts. It is expected to obtain a similar bone growth effect by using the magnetic field generator. More specifically, jawbone around a site where the implant body is inserted is magnetically stimulated in a magnetic field formed by the magnetic field generator. This promotes growth of osteoblasts of the magnetically stimulated jawbone, and growth of the jawbone is expected to be promoted. Hence, it is very likely that using this magnetic field generator reduces the duration of osseointegration.

As described above, the implant device includes the implant body and the magnetic field generator mountable to and removable from the implant body. Upon the completion of osseointegration, therefore, an abutment or the like constituting an upper structure can be coupled to the implant body after the magnetic field generator is removed from the implant body. The implant body is then ready for use as any conventional implants.

The denture base of the denture for implant protection has the protective concave portion and the magnetic member.

The protective concave portion can accommodate therein a part of the implant device protruding from jawbone in a non-contact manner, preventing the implant device from undergoing any force applied by the denture for implant protection. This imparts a postural stability to the implant device, ensuring that osseointegration is efficiently performed between the implant body and jawbone.

The magnetic member is magnetically connected to the magnetic field generator, and a magnetic field is formed around the magnetic member. The magnetic member helps to broaden an area where the magnetic field is formed, subjecting a broader area of jawbone around the implant body to magnetic stimulation. Then, growth of jawbone is efficiently and effectively enhanced, and the duration of osseointegration can be further reduced.

As described thus far, the magnetic field generator, and the implant device and the denture for implant protection using the magnetic field generator are highly anticipated to promote growth of jawbone and consequently reduce the duration of osseointegration.

### BRIEF DESCRIPTION OF THE DRAWINGS

Fig. 1 is a sectional view of an implant device and a denture for implant protection according to Example 1.
Fig. 2 is a sectional view of an implant body according to Example 1.
Fig. 3 is a sectional view of a magnetic field generator according to Example 1.
Fig. 4 is a distribution chart of a magnetic field formed by the magnetic field generator according to Example 1.
Fig. 5 is a sectional view of an implant device and a denture for implant protection according to Example 2.
Fig. 6 is a sectional view of a magnetic field generator according to Example 2.
Fig. 7 is a sectional view of an implant device and a denture for implant protection according to Example 3.
Fig. 8 is a sectional view of an implant body according to Example 3.
Fig. 9 is a sectional view of a magnetic field generator according to Example 3.
Fig. 10 is a sectional view of an implant device and a denture for implant protection according to Example 4.
Figs. 11(a) and 11(b) are sectional views of structural elements of the magnetic field generator according to Example 4: Fig. 11 (a) shows a magnet body and Fig. 11 (b) shows a keeper.

### EMBODIMENTS OF THE INVENTION

The magnetic field generator may include an insert portion to be inserted in a concave portion formed in the implant body when the magnetic field generator is mounted to the implant body, the insert portion may include at least one magnetic material member, and a magnetic circuit may be formed by the magnet body in the insert portion. In this case, the insert portion is inserted in the concave portion of the implant body, the magnet body forms magnetic poles in the insert portion, and a magnetic field is thereby formed around the implant body. This magnetic field applies magnetic stimulation to jawbone, promoting growth of the jawbone.

Preferably, the concave portion is formed as far as a position in the implant body closer to its edge, and the insert portion is inserted into the concave portion as far as a tip-end side position of the concave portion. In this case, a magnetic field can be formed in a larger area.

Magnetic materials are magnetizable substances, including soft magnetic materials exerting small coercive forces, and hard magnetic materials exerting large coercive forces. An example of the soft magnetic materials is a soft magnetic stainless steel, and an example of the hard magnetic materials is a permanent magnet.

At least one magnet body may be located at a position on an outer side in an axial direction of the implant body than an end part thereof where an opening of the concave portion is formed. According to this technical feature, the magnet body can be provided on the outer side of the implant body. In this case, the magnet body to be used can be made bigger than the one provided in the inner side of the implant body. As a result, a powerful magnetic field can be formed in a large area by the magnetic field generator. The axial direction of the implant is coincident with the longitudinal direction of the implant.

The insert portion and the magnet body may be detachably connected to each other by a magnetic force of the magnet body. According to this technical feature, the magnet body attracts the insert portion by magnetic attractive force. This facilitates locating the magnet body at a predetermined position and removing the located magnet body without a need to use a tool or the like.

Upon the completion of osseointegration, the insert portion and the implant body may continue to be available to fix the denture. The denture is furnished with an intra-denture magnetic assembly that exerts magnetic attractive force. This denture is fixable when the intra-denture magnetic assembly attracts the insert portion.

At least a part of the insert portion may include the magnet body. According to this technical feature, the permanent magnet can be situated at a position closer to integrated parts of the implant body and jawbone. This certainly forms a magnetic field in the parts of jawbone integrated with the implant body, more effectively promoting growth of the jawbone.

In the denture for implant protection, the magnetic member may include magnetic particles dispersed in the denture base. This technical feature is advantageous in that the magnetic particles can be dispersed in a broad area of the denture base. The dispersed magnetic particles and the magnetic field generator are magnetically connected to each other, and a magnetic field is accordingly formed around the implant body. This allows magnetic stimulation to be applied to a broader area of jawbone around the implant body, more effectively promoting growth of the jawbone.

### EXAMPLES

### (Example 1)

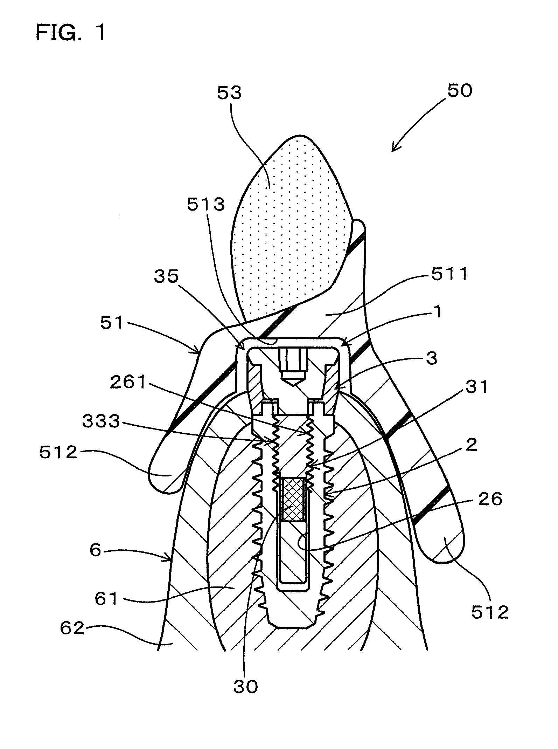

A magnetic field generator and an implant device according to an example are hereinafter described referring to Figs. 1 to 3.

As illustrated in Fig. 1, a magnetic field generator 3 is attachable to an implant body 2 formed from a non-magnetic material. The magnetic field generator 3 is inserted in jawbone 61. The magnetic field generator 3 includes a magnet body 30 that generates magnetism. The magnetic field generator 3 is mountable to and removable from the implant body 2. The magnetic field generator 3 mounted to the implant body 2 forms a magnetic field around the implant body 2.

A more detailed description is hereinafter given.

As illustrated in Fig. 1, an implant device 1 according to this example is used during the period of osseointegration to connect the jawbone 61 with the implant body 2 that fixedly supports a denture (not illustrated) to alveolar ridge 6 after the implant body 2 is inserted in the jawbone 61.

As illustrated in Fig. 1, the implant device 1 includes the implant body 2, and the magnetic field generator 3 mountable to and removable from the implant body 2.

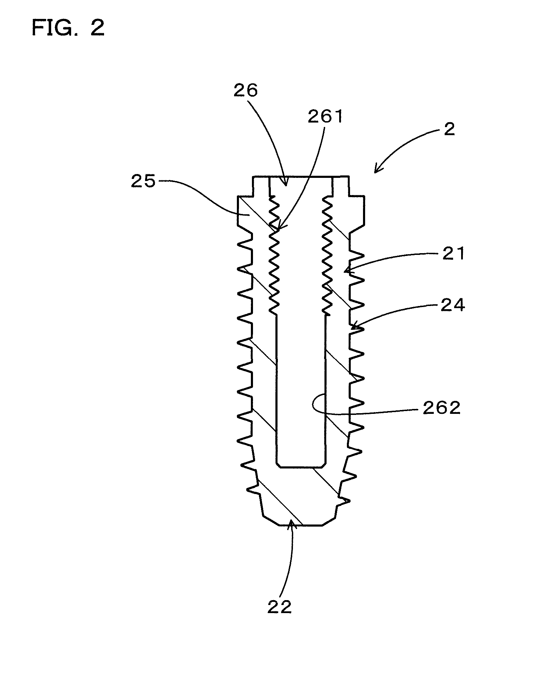

As illustrated in Fig. 2, the implant body 2 is formed from a titanium alloy and has a roughened surface with very fine projections and dents formed thereon. Such a roughened surface serves to firmly connect the implant body 2 to the jawbone 61. The roughened surface may be formed by one selected from various techniques, for example, shot blasting and etching using strong acids. An outer peripheral surface of the implant body 2 may be coated with hydroxyapatite. This further helps to reduce the duration of osseointegration. The implant body 2 has a closed-bottom cylindrical shape. The implant body 2 has a cylindrical implant cylinder 21 and an implant bottom 22 sealing a tip-end side opening of the implant cylinder 21.

As illustrated in Fig. 2, the implant body 2 has, on its outer peripheral surface, a threaded portion to be inserted 24 having a thread and an implant flange 25 formed on a base-end side of the threaded portion to be inserted 24.

The threaded portion to be inserted 24 is formed in a part of the implant body 2 from a tip-end to vicinity of a base end part thereof. The implant flange 25 is extending from a base-end part to an outer-peripheral side of the threaded portion to be inserted 24.

As illustrated in Fig. 2, the implant body 2 has a concave portion 26 formed in the inner periphery of the implant cylinder 21. The concave portion 26 has an internal thread portion 261 and an insert locating portion 262 formed at a part of the concave portion 26 closer to the implant bottom 22 than the internal thread portion 261.

The internal thread portion 261 is formed in a part of the implant cylinder 21 from vicinity of a base-end side opening to nearly an axially central position of the implant cylinder 21.

The insert locating portion 262 forms a cylindrical surface having a uniform inner diameter and is provided between the internal thread portion 261 and the implant bottom 22.

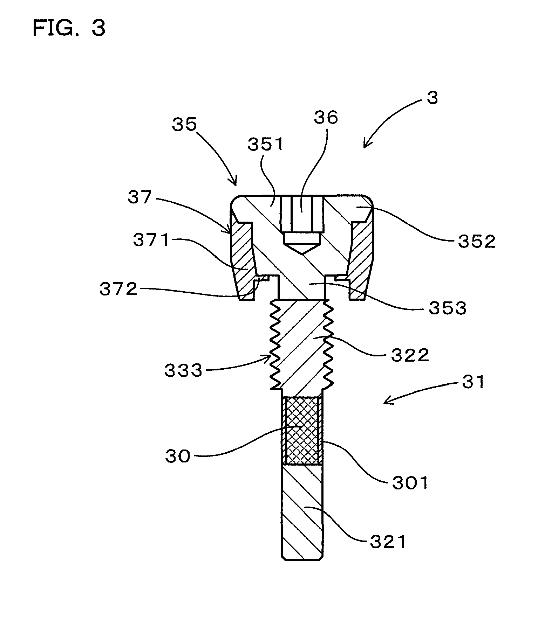

As illustrated in Figs. 1 and 3, the magnetic field generator 3 removably mounted to the concave portion 26 of the implant body 2 has an insert portion 31 to be inserted in the concave portion 26, and a head portion 35 formed in a base-end part of the insert portion 31.

The insert portion 31 has a magnet body 30, insert members 321 and 322 with the magnet body 30 axially held therebetween, and a casing member 301 covering the side surface of the magnet body 30.

As illustrated in Fig. 3, the magnet body 30 has a columnar permanent magnet, and the casing member 301 covers the side surface of the magnet body 30 in a direction orthogonal to the axial direction of the magnet body 30. The magnet body 30 is axially magnetized, and magnetic poles are formed on both axial ends of the magnet body 30. The magnet body 30 may be magnetized in a direction other than the axial direction. However, axially magnetizing the magnet body 30 may further broaden an area where the magnetic field is formed.

The casing member 301 has a cylindrical shape having an inner diameter corresponding to the outer diameter of the magnet body 30, and the magnet body 30 is inserted and located in the inner periphery of the casing member 301.

As illustrated in Fig. 3, the insert member 321 and the insert member 322 are respectively a tip-end side insert member 321 located closer to the tip-end side than the magnet body 30, and a base-end side insert member 322 located closer to the base-end side than the magnet body 30.

The tip-end side insert member 321 is formed from a soft magnetic stainless steel in a columnar shape that allows this member to be inserted in the concave portion 26 of the implant body 2.

As illustrated in Fig. 3, the base-end side insert member 322 is formed from a soft magnetic stainless steel in a columnar shape and has threads formed on its outer peripheral side surface. The base-end side insert member 322 is engageable with the internal thread portion 261 of the concave portion 26.

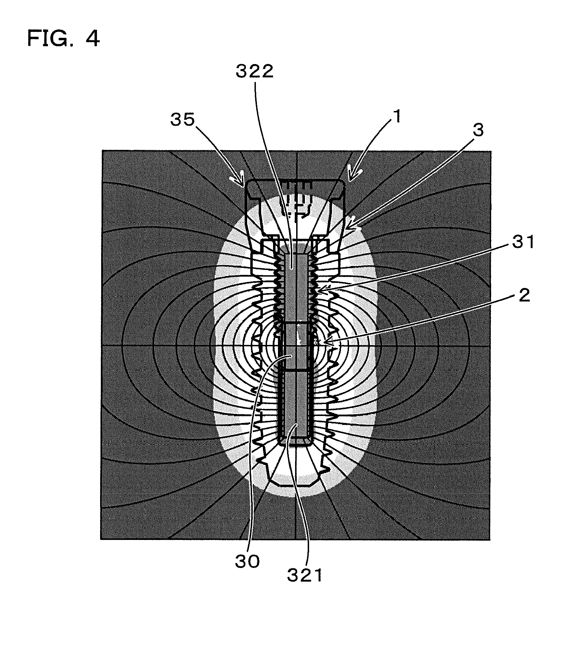

The magnet body 30 and the insert members 321 and 322 are integrally formed. When the magnetic field generator 3 is mounted to the implant body 2, the magnet body 30 is located at nearly an axially central position of the implant cylinder 21. The insert members 321 and 322 integral with the magnet body 30 are magnetized by the magnet body 30, and magnetic poles are respectively formed in the insert members 321 and 322. This forms an open magnetic circuit, as illustrated in Fig. 4, from a side-surface part of the base-end side insert member 322 to a side-surface part of the tip-end side insert member 321, in which lines of magnetic force pass through the jawbone 61 and the implant body 2 located around the insert portion. The lines of magnetic force forming the open magnetic circuit may be directed opposite to the before-mentioned direction.

In this example, the magnetic body 30 and the insert members 321 and 322 integral with one another constitute the insert portion 31. However, the insert portion 31 may be structured otherwise to an extent that ensures magnetic connection between the magnetic body 30 and the insert members 321 and 322. The magnetic poles of the insert portion 31 may be formed by the use of the insert members 321 and 322 as described in this example, or the magnet body 30 alone may constitute the whole insert portion 31 and form magnetic poles.

As illustrated in Fig. 3, the head portion 35 is provided on the base-end side of the base-end side insert member 322 of the insert portion 31. The head portion 35 has a head body 351 formed from a non-magnetic material, and a gingiva abutting portion 37 provided around the head body 351.

As illustrated in Fig. 3, the head body 351 has a substantially columnar shape. The head body 351 has a head flange 352 extending from a base-end side toward an outer-peripheral side thereof, and a columnar head coupling portion 353 protruding from a tip-end side end surface thereof. At an end surface on the base-end side of the head body 351 is formed with an engaging hole 36 having a hexagonal shape in axial view that engageably accepts a hexagonal wrench.

The base-end side insert member 322 of the insert portion 31 is joined to an edge surface of the head coupling portion 353.

As illustrated in Fig. 3, the gingiva abutting portion 37 is formed from a titanium alloy in a substantially cylindrical shape. The gingiva abutting portion 37 has a substantially cylindrical abutting body 371, and an annular inner peripheral flange 372 extending from an inner peripheral surface toward a further inner side of the abutting portion 371.

As illustrated in Fig. 3, the head body 351, from the head flange 352 to a tip-end side thereof, is inserted in a base-end side opening of the abutting body 371. As illustrated in Fig. 1, a base end part of the implant body 2 is inserted in a tip-end side opening of the abutting body 371 when the implant body 2 is coupled thereto. The abutting body 371 and the implant body 2 are thereby tightly fitted to each other.

The inner peripheral flange 372 is formed to be located correspondingly to the edge side end surface of the head body 351 when the gingiva abutting portion 37 is mounted on the head body 351.

As to steps of burying the implant device 1 of this example in the alveolar ridge 6, to start with, a hole is formed in the gingiva 62 of the alveolar ridge 6, and a threaded hole is formed in the jawbone 61. Then, the implant body 2 is screwed into the threaded hole formed in the jawbone, and the insert portion 31 of the magnetic field generator 3 is inserted in the concave portion 26 of the implant body 2. The implant body 2 and the magnetic field generator 3 are detachably fixed by engaging the internal thread portion 261 and an external thread portion 333 with each other. At this time, the implant body 2 and the magnetic field generator 3 are tightly secured to each other without any interval therebetween.

After that, the jawbone 61 grows, filling in very fine projections and dents formed on the surface of the implant body 2. Then, the jawbone 61 and the implant body 2 are firmly connected to each other. During that while, the head portion 35 of the magnetic field generator 3 is acting as a healing abutment, allowing the formation to proceed without closing the hole of the gingiva 62.

As illustrated in Fig. 1, a denture for implant protection 50 is placed and kept in the alveolar ridge 6 with the implant device 1 inserted therein until osseointegration is completed.

The denture for implant protection 50 has a denture base 51 that covers the alveolar ridge 6, and an artificial tooth 53 formed on the denture base 51.

As illustrated in Fig. 1, the denture base 51 has a summit portion 511 located in an upper part of the alveolar ridge 6, and a side wall portion 512 extending from both ends of the summit portion 511. A protective concave portion 513 is provided on the back-surface side of the summit portion 511 of the denture base 51. The protective concave portion 513 accommodates therein the head portion 35 of the implant device 1 protruding from the alveolar ridge 6 in a non-contact manner.

After the jawbone 61 and the implant body 2 are firmly connected to each other by osseointegration, a denture fixing member (not illustrated) may be attached to the implant body 2 so that the denture having a denture-side fixing member (not illustrated) may be securely fixed in the alveolar ridge 6.

Next, effects of this example are described.

In this example, the magnetic field generator 3 forms a magnetic field around the implant body 2. The magnetic field thus generated is highly expected to reduce the duration of osseointegration.

A well-accepted practice for effective bone growth in the field of orthopedics is to impart magnetic stimulation to osteoblasts through a magnetic field to promote growth of the osteoblasts. Therefore, it is expected to obtain a similar bone growth effect by using the magnetic field generator 3. More specifically, the jawbone 61 around a site where the implant body 2 is inserted is magnetically stimulated in a magnetic field formed by the magnetic field generator 3. This promotes growth of osteoblasts of the magnetically stimulated jawbone 61, and growth of the jawbone 61 is expected to be promoted. Thus, using the magnetic field generator 3 is very likely to reduce the duration of osseointegration.

As described earlier, the implant device 1 includes the implant body 2 and the magnetic field generator 3 mountable to and removable from the implant body 2. Upon the completion of osseointegration, therefore, an abutment or the like constituting an upper structure can be coupled to the implant body 2 after the magnetic field generator 3 is removed from the implant body 2. The implant body 2 is then ready for use as a conventional implant.

In the implant device 1, the insert portion 31 to be inserted in the concave portion 26 of the implant body 2 has the soft magnetic insert members 321 and 322, and the magnet body 30 connected to these insert members 321 and 322. When the magnet body 30 and the soft magnetic insert members 321 and 322 are magnetically connected to one other, a magnetic field can be formed around the insert portion 31. The insert portion 31 inserted in the concave portion 26 of the implant body 2 consequently forms a magnetic field around the implant body 2, promoting growth of the jawbone 61.

The magnet body 30 is contained in the insert portion 31, and the permanent magnet is accordingly situated at a position closer to the integrated parts of the implant body 2 and the jawbone 61. This certainly forms a magnetic field in the parts of jawbone 61 integrated with the implant body 2, more effectively promoting growth of the jawbone 61.

As described thus far, the magnetic field generator 3 and the implant device 1 using the same are highly anticipated to promote growth of the jawbone 61 and consequently reduce the duration of osseointegration.

### (Example 2)

This example presents a modified structure of the magnetic field generator of Example 1.

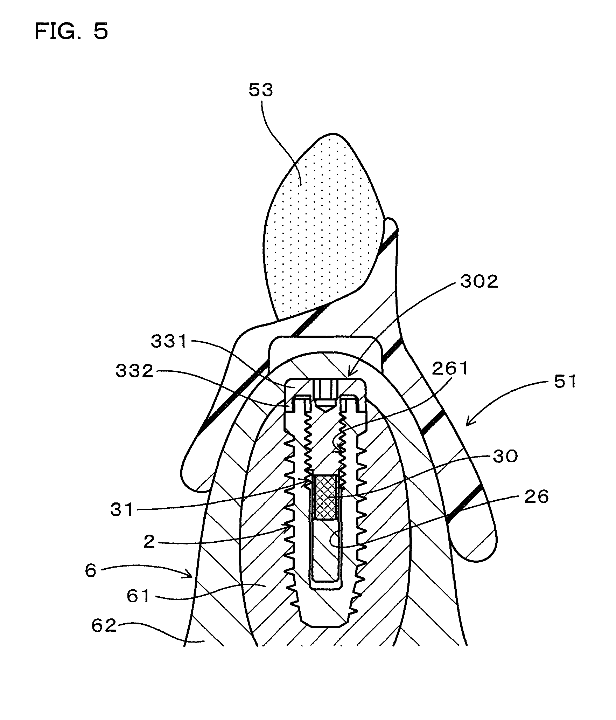

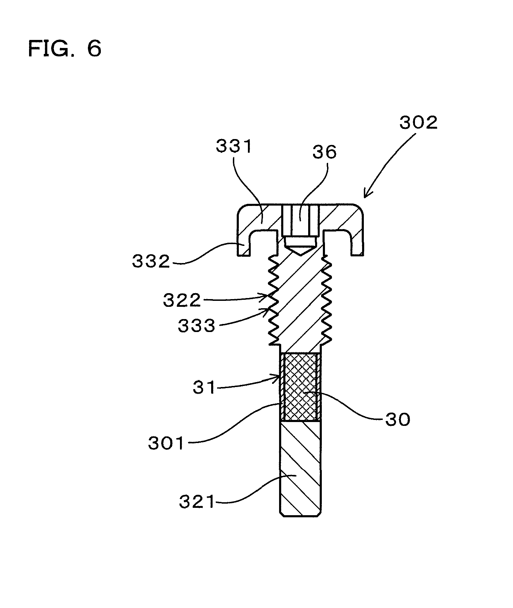

As illustrated in Figs. 5 and 6, a magnetic field generator 302 has an insert portion 31 to be inserted in the concave portion 26 of the implant body 2.

As illustrated in Fig. 6, the insert portion 31 has a magnetic body 30 including a permanent magnet, a casing member 301 covering the outer periphery of the magnet body 30, a tip-end side insert member 321 provided on a tip-end side of the magnet body 30, and a base-end side insert member 322 provided on a base-end side of the magnet body 30. The magnet body 30, casing member 301, and tip-end side insert member 321 are structured similarly to those described in Example 1.

As illustrated in Fig. 6, the base-end side insert member 322 is formed in a columnar shape and inserted in the concave portion 26 of the implant body 2. A disc-shaped lid portion 331 is formed at a base-end part of the base-end side insert member 322. Formed at an outer peripheral edge of the lid portion 331 is an outer peripheral cylinder 332 extending toward an edge side of the base-end side insert member 322.

The base-end side insert member 322 has an external thread portion 333 with threads formed on its outer peripheral side surface. The external thread portion 333 is engageable with an internal thread portion 261 formed in the concave portion 26 of the implant body 2.

An engaging hole 36 is formed at a substantially central position on an end surface on the base-end side of the lid portion 331.

The lid portion 331 has an axial linear dimension of approximately 1 mm, which is smaller than the axial linear dimension of the head portion 35 of Example 1.

Any other technical features are similar to those of Example 1.

As described above, the implant device 1 of this example is structured such that the lid portion 331 of the base-end side insert member 322 has an axially smaller dimension. As a result of this, protrusion of the implant device 1 from the jawbone 61 is reduced. When the gingiva 62 is sutured after the implant device 1 is inserted in the alveolar ridge 6, therefore, the inserted implant device 1 may be unexposed from the alveolar ridge 6.

In this case, a hole is formed in the gingiva 62 in a later stage of or upon completion of osseointegration, and the magnetic field generator 3 or healing abutment described in Example 1 is mounted to the implant body 2. This allows the formation to proceed without closing the hole formed in the gingiva 62.

Any other effects are similar to those obtained in Example 1.

### (Example 3)

This example presents modified structures of the magnetic field generator, implant device, and denture for implant protection according to Example 1.

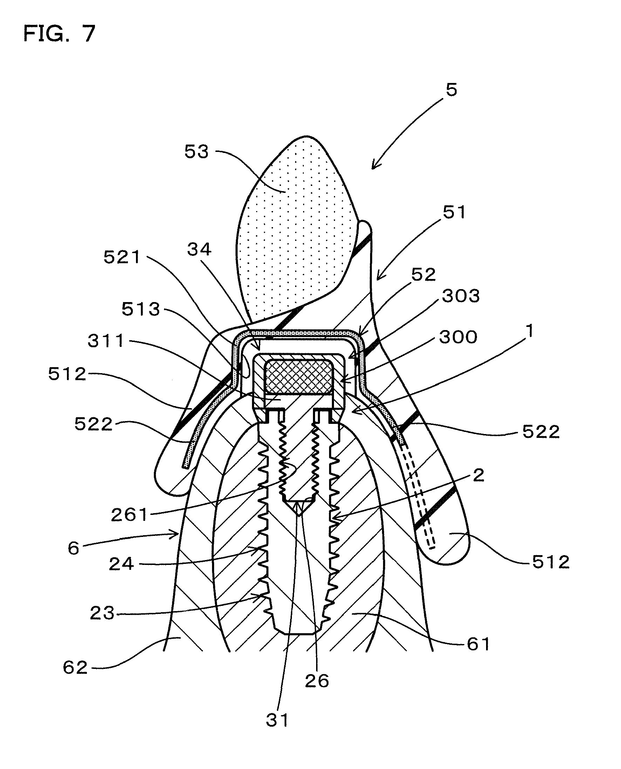

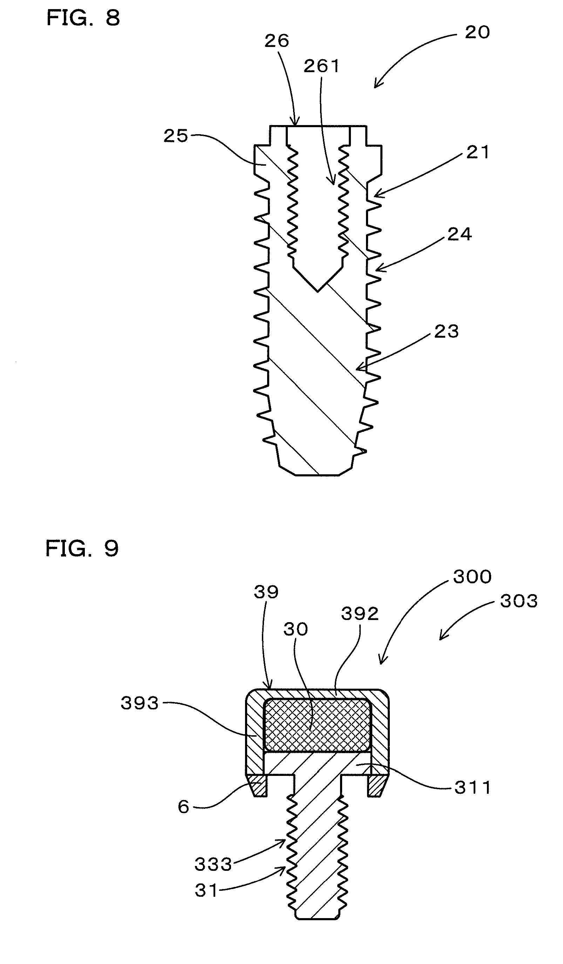

As illustrated in Figs. 7 and 8, an implant body 20 has a cylindrical implant cylinder 21 and a columnar implant solid-core portion 23 sealing a tip-end side opening of the implant cylinder 21. When axially viewed, the implant cylinder 21 constitutes a base-end side substantially half of the implant body 20, and the implant solid-core portion 23 constitutes the other half of the implant body 20.

A concave portion 26 formed by the inner periphery of the implant cylinder 21 constitutes an internal thread portion 261 with threads formed on its inner peripheral surface.

As illustrated in Figs. 7 and 9, a magnetic field generator 303 includes an insert portion 31 formed from a soft magnetic stainless steel, and a magnetic assembly 300 integral with the insert portion 31.

The insert portion 31 is formed from a soft magnetic stainless steel in a columnar shape. The insert portion 31 has an external thread portion 333 with threads formed on its outer peripheral side surface. The external thread portion 333 is engageable with the internal thread portion 261 formed in the concave portion 26 of the implant body 20.

The disc-shaped magnetic field generator 303 magnetic field generator 303 covering an opening of the magnetic assembly 300 is integrally formed at a base-end part of the insert portion 31.

As illustrated in Fig. 9, the magnetic assembly 300 is integrally formed on a base-end side surface of the insert portion 31.

The magnetic assembly 300 has a magnet body 30 including a permanent magnet, a casing 39 provided around the magnet body 30, and a casing 39 provided at an opening end part of the casing 39.

The magnet body 30 has a substantially columnar shape, and its outer diameter is equal to the outer diameter of the lid portion 311. The magnet body 30 is axially magnetized, and magnetic poles are thereby formed on axial both ends of the magnet body 30. The magnet body 30 may be magnetized in a direction other than the axial direction. However, axially magnetizing the magnet body 30 further broadens an area where the magnetic field is formed.

As illustrated in Fig. 9, the casing 39 is formed from a non-magnetic stainless steel. The casing 39 has a casing upper surface portion 392 formed in an upper part of the magnet body 30, and a casing cylindrical portion 393 formed on the outer periphery of the magnet body 30.

The casing cylindrical portion 393 is formed in a cylindrical shape from an outer peripheral edge part of the disc-shaped casing upper surface portion 392 toward the magnet body 30. The casing cylindrical portion 393 has an axial linear dimension substantially equal to a linear dimension obtained by summing the axial dimensions of the magnet body 30 and the lid portion 311.

As illustrated in Fig. 9, a ring member 6 having a substantially annular shape is provided at an opening end part of the casing cylindrical portion 393 containing therein the magnet body 30 and the lid portion 311. The ring member 6 is acting as a spacer that clears any gap between the magnetic field generator 303 and the implant body 2. In this example, the ring member 6 is integrally fixed with the casing 39. Instead, the ring member 6 and the casing 39 may be provided independently as separate members.

As illustrated in Fig. 9, the casing 39, magnet body 30, and insert portion 31 are structured such that the magnet body 30 and the lid portion 311 are located inside the casing 39, and the casing 39 and the lid portion 311 are then fixedly secured to each other. A means for fixing the casing 39 and the lid portion 311 to each other may be welding or press-fitting.

The insert portion 31 magnetically connected to the magnet body 30 is magnetized by the magnet body 30, and magnetic poles are thereby formed in the axial direction of the insert portion 31. This forms an open magnetic circuit, from a base-end side end surface of the magnetic assembly 300 to the insert portion 31, wherein lines of magnetic force pass through the jawbone 61 and the implant body 2 located around the insert portion 31.

As illustrated in Fig. 7, when the magnetic field generator 303 is provided in the implant body 2, the magnet body 30 is located at a position on the outer side in the axial direction of the implant body 2 than an end part thereof where the opening of the concave portion 26 is formed, in other words, at a position on the opposite side of the tip-end part of the implant body 2.

As illustrated in Fig. 7, the denture for implant protection 5 mounted on the alveolar ridge 6 with the implant device 1 inserted therein has a magnetic member 52 inside the denture base 51 so as to cover a part of the alveolar ridge 6 where the implant device 1 is planted.

Provided on the back-surface side of the summit portion 511 of the denture base 51 is a protective concave portion 513 that, in a non-contact manner, accommodates therein the magnetic assembly 300 of the implant device 1 protruding from the alveolar ridge 6.

The magnetic member 52 provided in the denture base 51 is formed by bending a soft magnetic stainless steel plate. The magnetic member 52 has an upper transmitting portion 521 having a substantially U shape in cross section, and a lateral transmitting portion 522. The upper transmitting portion 521 is formed around the magnetic assembly 300 of the implant device 1 and along the inner peripheral surface of the protective concave portion 513. The lateral transmitting portion 522 is extending along the alveolar ridge 6 from both ends of the upper transmitting portion 521.

Any other technical features are similar to those of Example 1.

In the magnetic field generator 303 of this example, the magnet body 30 of the magnetic assembly 300 is located at a position on the outer side in the axial direction of the implant body 2 than an end part thereof where the opening of the concave portion 26 is formed. According to this technical feature, the magnet body 30 can be provided on the outer side of the implant body 2. Thereby, a size of the magnet body 30 can be made bigger than the case of providing the magnet body 30 in the inner side of the implant body. As a result, a powerful magnetic field can be formed in a large area by the magnetic field generator 303. The magnetic field generator 303 of this example is further advantageous in that the structural components can be simplified, leading to a better productivity.

The denture base 51 of the denture for implant protection 5 has the protective concave portion 513 and the magnetic member 52.

As described above, the protective concave portion 513 can accommodate therein a part of the implant device 1 protruding from the jawbone 61 in a non-contact manner, preventing the implant device 1 from undergoing any force applied by the denture for implant protection 5. This imparts a postural stability to the implant device 1, ensuring that osseointegration is efficiently performed between the implant body 202 and the jawbone 61.

The magnetic member 52 is magnetically connected to the magnetic field generator 303, and a magnetic field is formed around the magnetic member 52. Then, the magnetic member 52 serves to expand an area where the magnetic field is formed, subjecting a broader area of the jawbone 61 around the implant body 202 to magnetic stimulation. Then, growth of the jawbone 61 is efficiently and effectively enhanced, and the duration of osseointegration can be further reduced. Any other effects are similar to those obtained in Example 1.

### (Example 4)

This example presents modified structures of the implant device and the denture for implant protection according to Example 3.

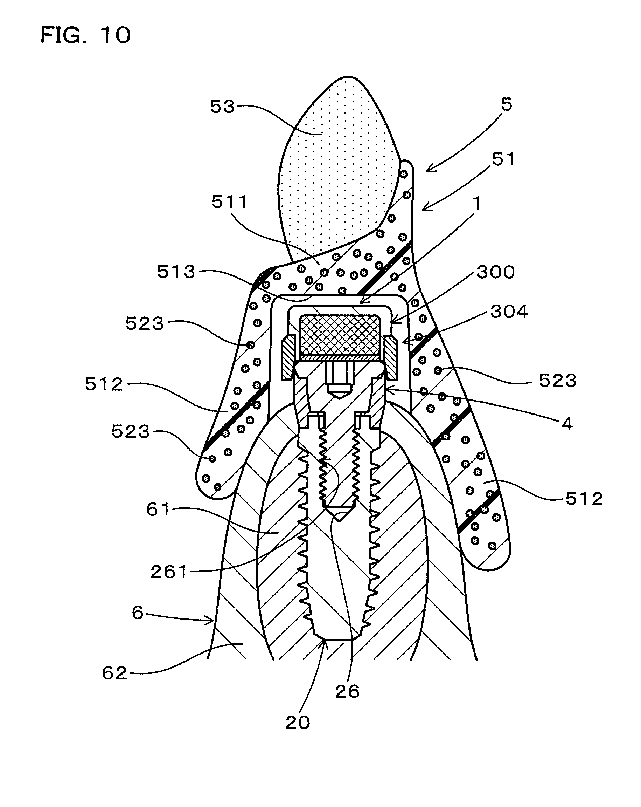

As illustrated in Fig. 10, an implant device 1 includes an implant body 20 similar to that of Example 3, and a magnetic field generator 304 mounted to the concave portion 26 of the implant body 20.

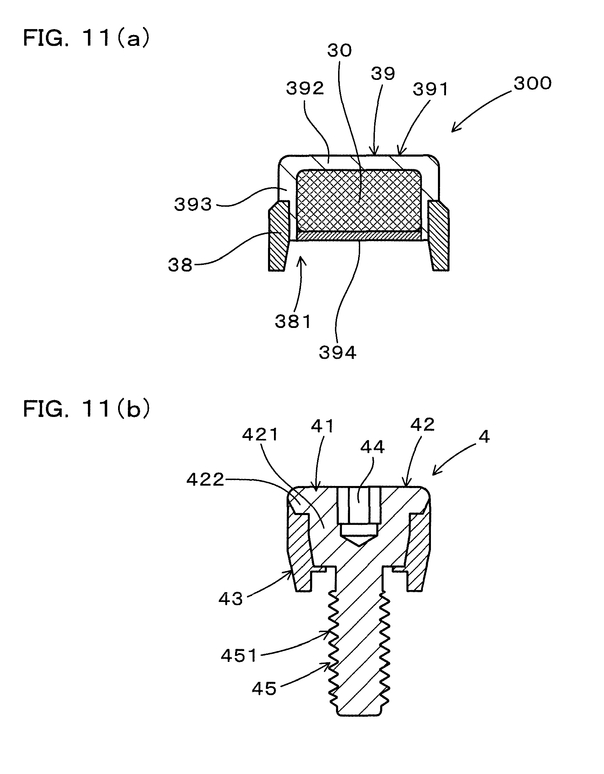

As illustrated in Figs. 10 and 11, the magnetic field generator 304 has a magnetic assembly 300 containing therein a magnet body 30, and a keeper 4 fixedly attractable by the magnetic force of the magnet body 30.

As illustrated in Fig. 11 (a), the magnetic assembly 300 has a magnet body 30 including a columnar permanent magnet, a casing 39 that accommodates therein the magnet body 30, and a sleeve 38 provided on an outer-peripheral side of the casing 39.

The casing 39 has a casing body 391 having a closed-bottom cylindrical shape, and a lid 394 that covers an opening end part of the casing body 391.

As illustrated in Fig. 11 (a), the casing body 391 is formed from a non-magnetic stainless steel. The casing body 391 has a casing upper surface portion 392 formed in an upper part of the magnet body 30, and a casing cylindrical portion 393 formed on an outer peripheral edge part of the casing upper surface portion 392. The casing cylindrical portion 393 has an axial linear dimension substantially equal to an axial linear dimension of the magnet body 30 with the lid 394 attached thereto.

The lid 394 is formed from a soft magnetic stainless steel in a disc shape, and has an outer diameter corresponding to the inner diameter of the casing cylindrical portion 393.

The magnetic assembly 300 is structured such that the magnet body 30 is located inside the casing body 391, and the lid 394 is press-fitted to the inner side of the opening end part of the casing body 391, so that the magnet body 30 is sealed in the casing 39. A side surface of the casing 39 provided with the lid 394 serves as a surface to which the keeper 4 is attracted by the magnetic force of the magnet body 30.

As illustrated in Fig. 11 (a), the sleeve 38 has a substantially cylindrical overall shape. One opening end part of the sleeve 38 is situated so as to cover an outer peripheral surface of the casing body 391 on the opening-end side. The other opening end part of the sleeve 38 is protruding farther than the opening end part of the casing body 391, forming a keeper locating concave portion 381 that can accommodate therein the keeper 4 when the magnet body 30 is attached to the keeper 4.

As illustrated in Fig. 11 (b), the keeper 4 has a keeper body 41 formed from a magnetic material member, and a gingiva abutting portion 43 provided on an outer peripheral side of the keeper body 41.

The keeper body 41 has an insert portion 45 to be inserted in the concave portion 26 of the implant body 20, and a head portion 42 protruding from the alveolar ridge 6.

As illustrated in Fig. 11 (b), the insert portion 45 has a columnar shape. The insert portion 45 has an external thread portion 451 with threads formed on its outer peripheral side surface. The external thread portion 451 is engageable with the internal thread portion 261 formed in the concave portion 26.

The head portion 42 is formed on a base-end side of the insert portion 45. The head portion 42 has a head body 421 integrally formed with the insert portion 45, and a gingiva abutting portion 43 provided on the outer periphery of the head body 421.

As illustrated in Fig. 11 (b), the head body 421 has a substantially columnar shape. The head body 421 has a head flange 422 extending from a base end part toward an outer-peripheral side thereof. Formed at an end surface on the base-end side of the head body 421 is an engaging hole 44 having a hexagonal shape in axial view that engageably accepts a hexagonal wrench. The end surface on the base-end side of the head body 421 constitutes a surface to be attracted to the magnet body 30.

As illustrated in Fig. 10, when the head portion 42 of the keeper 4 is inserted in the keeper locating concave portion 381 of the magnetic assembly 300, the surfaces-to-be-attracted of the magnetic assembly 300 and the keeper 4 are attracted to each other by the magnetic force generated by the magnet body 30. As a result, the magnetic assembly 300 and the keeper 4 are fixedly secured to each other.

As illustrated in Fig. 10, magnetic particles 523 acting as the magnetic member 52 are dispersed in the denture base 51 of the denture for implant protection 5 mounted on the alveolar ridge 6 with the implant device 1 inserted therein. Examples of the magnetic particles 523 are pulverized permanent magnets and plastic magnets.

Any other technical features are similar to those of Example 1.

According to the implant device 1 of this example, the magnetic assembly 300 attracts the keeper 4 by magnetic attractive force. This facilitates locating the magnet body 30 at a predetermined position and removing the located magnet body 30 without a need to use a tool or the like.

Upon the completion of osseointegration, the keeper 4 and the implant body 2 may continue to be available to fix the denture. The denture is furnished with an intra-denture magnetic assembly that exerts magnetic attractive force. The denture is fixable through adsorption of the intra-denture magnetic assembly to the keeper 4.

In the denture for implant protection 5, the magnetic particles 523 dispersed in the denture base 51 are used as the magnetic member 52. This technical feature is advantageous in that the magnetic particles 523 are dispersible in a broad area of the denture base 51. The magnetic field formed by the magnetic field generator 304 is broadened through the dispersed magnetic particles 523. As a result, an area of the jawbone 61 subjected to magnetic stimulation is also broadened.

Examples of the magnetic particles described in this example are pulverized permanent magnets and plastic magnets. Using these magnetic materials enables the formation of a magnetic field around the implant body even when the denture for implant protection is solely used, and the effect of reducing the duration of osseointegration can be expected. When the magnetic particles are used in combination with the magnetic field generator, the magnetic field may be formed by both the pulverized permanent magnet and the magnet body, so that the magnetic field may be formed certainly in a larger range. This further enhances the prospect of even shorter duration of osseointegration.

Any other effects are similar to those obtained in Example 1.

The magnet body of the magnetic field generator used in Examples 1 to 4 is a permanent magnet. Instead, an electromagnetic device may be used.

The insert portion may be partly formed from a magnetic material member. Preferably, the whole insert portion is formed from a magnetic material member. Preferably, the insert member is formed from the soft magnetic material and the magnet body as described in Examples 1 and 2, the soft magnetic material alone as described in Examples 3 and 4, or the magnet body alone. The insert portion at least partly including the magnet body is preferably structured similarly to the insert portions described in Examples 1 and 2. According to such an insert portion, the magnetic field is formed in a larger range, which leads to high expectations for even shorter duration of osseointegration.

The magnetic field generator may have a plurality of magnet bodies. For example, the magnetic field generator may have two magnet bodies; a magnet body contained in the insert portion as described in Examples 1 and 2, and a magnet body located at a position on the outer side of the implant body than the end part thereof where the opening of the concave portion is formed as described in one of Examples 3 and 4. The magnetic field is accordingly formed in a larger range, which leads to high expectations for even shorter duration of osseointegration.

## Claims

1. A magnetic field generator attachable to an implant body formed from a non-magnetic material to be inserted in jawbone comprising at least one magnet body that generates magnetism, wherein
the magnetic field generator is mountable to and removable from the implant body, and
the magnetic field generator in a state of being mounted to the implant body forms a magnetic field around the implant body.

2. The magnetic field generator according to claim 1, comprising an insert portion to be inserted in a concave portion formed in the implant body when the magnetic field generator is mounted to the implant body, wherein
the insert portion comprises at least one magnetic material member, and
a magnetic circuit is formed by the magnet body in the insert portion.

3. The magnetic field generator according to claim 2, wherein at least one magnet body is located at a position on an outer side in an axial direction of the implant body than an end part thereof where an opening of the concave portion is formed.

4. The magnetic field generator according to claim 3, wherein the insert portion is made from a soft magnetic material, and the insert portion and the magnet body are detachably connected to each other.

5. The magnetic field generator according to claim 2 or 3, wherein at least a part of the insert portion comprises the magnet body.

6. An implant device, comprising:
the magnetic field generator according to any one of claims 1 to 5; and
an implant body formed from a non-magnetic material to be inserted in jawbone, wherein
the magnetic field generator is mountable to and removable from the implant body.

7. A denture for implant protection mounted on alveolar ridge mounted with the implant device according to claim 6, comprising:
a denture base for covering the alveolar ridge; and
an artificial tooth formed on the denture base, wherein
the denture base has a protective concave portion that, in a non-contact manner, accommodates therein a part of the implant device protruding from the jawbone, and
the denture base comprises a magnetic member that induces a magnetic field from the magnetic field generator.

8. The denture for implant protection according to claim 7, wherein the magnetic member includes magnetic particles dispersed in the denture base.
